# EUROPÄISCHE PATENTSCHRIFT

(11) **EP 4 247 343 B1**
(45) Veröffentlichungstag und Bekanntmachung des Hinweises auf die Patenterteilung: **06.08.2025**
(21) Anmeldenummer: 21815484.7
(22) Anmeldetag: 18.11.2021
(51) Int. Cl.: A61K 9/107, A61K 47/14, A61K 47/36, A61K 47/40, A61K 47/46, A61K 35/00, A61K 45/06, A61K 31/12, A61K 31/366, A61K 36/28, A61K 36/324, A61K 36/9066, A61K 31/375

(54) **MIZELLARES SYSTEM ZUM TRANSPORT VON WIRKSTOFFEN**
MICELLAR SYSTEM FOR TRANSPORTING ACTIVE INGREDIENTS
SYSTÈME MICELLAIRE POUR LE TRANSPORT D'INGRÉDIENTS ACTIFS

(30) Priorität: 20.11.2020 CH 14812020
(43) Veröffentlichungstag der Anmeldung: 27.09.2023
(73) Patentinhaber: Mivital AG, 9014 St. Gallen (CH)
(72) Erfinder: STRASSER, Daniel, 9200 Gossau (CH); WUNDERLI, Samuel, 8252 Schlatt (CH); STRASSER, Andrin A., 8050 Zürich (CH)
(74) Vertreter: E. Blum & Co. AG
(86) Internationale Anmeldenummer: PCT/EP2021/082216
(87) Internationale Veröffentlichungsnummer: WO 2022/106576

(56) Entgegenhaltungen:
- WO-A1-2010/022524
- CA-A1- 2 922 959
- VITANA-X: "Bleib gesund -stay healthy See more of Bleib gesund -stay healthy on Facebook Log In or Create New Account Log In", 24 March 2020 (2020-03-24), pages 1 - 1, XP055892223, Retrieved from the Internet <URL:https://www.facebook.com/106285677685114/photos/a.106285724351776/106287594351589/?type=3&is_lookaside=1> [retrieved on 20220215]
- VITANA-X: "PRODUKTÜBERSICHT", 1 January 2019 (2019-01-01), pages 1 - 23, XP055892218, Retrieved from the Internet <URL:https://jimdo-storage.global.ssl.fastly.net/file/6151fb83-43c8-4e34-b3bf-0d5657196d38/vitanax-products-german-DE-min.pdf>

## Beschreibung

### Gebiet der Erfindung

Die Erfindung betrifft Wirkstoffzusammensetzungen, die künstlich hergestellte, selbst agglomerierende, stabile Mizellen mit guten Transporteigenschaften für einen inkorporierten Wirkstoff umfassen, sowie ein Herstellungsverfahren für solche Mizellen.

### Hintergrund

Wirkstoffzusammensetzungen enthaltend einen einzigen Typ von Micellen sind an sich bekannt. Aus WO 2008/034273 sind Mizellen bekannt, die Wirkstoffe und Gummi Arabicum enthalten, aus WO 2008/138155 sind Mizellen bekannt, die Wurzelharze und gegebenenfalls Gummi Arabicum enthalten und aus WO 2010/022525 sind mizellare Zusammensetzungen bekannt, die einen oder mehrere Wirkstoffe, Glycerinfettsäureester und ggf. Gummi Arabicum, Wurzelharz oder Cyclodextrin enthalten. Die Produktserie Vitana-X beschreibt micellare Zusammensetzungen mit verbesserter Bioverfügbarkeit ausgewählter Wirkstoffe.

Aus CA 2922959 A1 sind Wirkstoffzusammensetzungen enthaltend zwei Typen von Micellen bekannt, die Glycerinester und/oder Polyelektrolyte enthalten können.

Wirkstoffzusammensetzungen mit hohem Gehalt an unterschiedlichen Wirkstoffen bereit zu stellen ist immer noch eine Herausforderung. Wirkstoffzusammensetzungen mit gezieltem und definiertem Freisetzungsprofil bereit zu stellen ist ebenfalls immer noch eine Herausforderung.

Ziel der vorliegenden Erfindung ist es deshalb, Wirkstoffzusammensetzungen bereitzustellen, die viel Wirkstoff aufnehmen und vorzugsweise oral verabreicht werden können. Ziel der vorliegenden Erfindung ist es auch, Wirkstoffzusammensetzungen bereit zu stellen, die Wirkstoff an einem vorbestimmten Ort (gerichtet) und / oder über eine vorbestimmte Zeit (release-Profil) freisetzen.

### Darstellung der Erfindung

Dieses Ziel wurde erreicht durch Bereitstellen von Mizellen enthaltenden Wirkstoffzusammensetzungen, sowie Verfahren zur Herstellung entsprechender Mizellen und Wirkstoffzusammensetzungen.

Erfindungsgemässe Wirkstoffzusammensetzungen enthalten mindestens zwei Typen von Mizellen, wobei jeder Mizellentyp neben mindestens einem Wirkstoff mindestens ein Oligo- und/oder Polysaccharid (in der Folge allgemein als Polyelektrolyte bezeichnet) und/oder mindestens einen Glycerinester umfasst, der ausgewählt ist aus Glycerinestern von Fettsäuren und Wurzelharzen. Nachfolgend soll die Erfindung in ihren verschiedenen Ausgestaltungen näher erläutert werden

### Micellen

Der Begriff Micellen ist bekannt. Micellen gemäss der vorliegenden Erfindung weisen einen Kern auf - dieser enthält vorwiegend den Wirkstoff - und eine einfache oder komplexe Hülle, diese weist Hilfsstoffe aus der Gruppe der Polyelektrolyten und / oder Glycerinester auf. Weitere Komponenten können im Kern oder in der Hülle zugegen sein. Sofern die Hülle Polyelektrolyten und Glycerinester aufweist, wird von einem "ersten Typ von Micellen" oder "komplexen Micellen" gesprochen. Sofern die Hülle Polyelektrolyten oder Glycerinester aufweist, wird von einem "zweiten Typ von Micellen" oder "einfachen Micellen" gesprochen.

Der mittlere geometrische Durchmesser erfindungsgemässer Mizellen beträgt 5 nm - 100 nm, besonders bevorzugt 10 nm - 30 nm (optische Messung, angegeben als d₉₀-Wert, d.h. im Quantil von 5 - 95, unabhängig von der Grössenverteilung). Dieser ist damit wesentlich kleiner als die Dimension der meisten menschlichen Zellen und kleiner als der geometrisch-optische Durchmesser der meisten Viren. Ohne sich an eine Theorie gebunden zu fühlen, wird davon ausgegangen, dass diese relativ kleinen Dimensionen der Mizellen im Vergleich zu menschlichen Gewebszellen die Abwesenheit einer Immunreaktion begünstigen. Die nanoskaligen Mizellen unterlaufen das Ansprechen des Immunsystems effizient.

Das Verhältnis der beiden Micellentypen untereinander kann in weiten Bereichen variieren und hängt bspw. von der Wahl des Wirkstoffes ab. Geeignete Verhältnisse von erstem Typ Micellen zu zweitem Typ Micellen liegen im Bereich 1:99 bis 99:1 (n/n) bevorzugt 10:90 bis 90:10, wie bspw. 2:1 bis 1:2. Der erste Micellentyp enthält neben Wirkstoff mindestens einen Polyelektrolyten und mindestens einen Glycerinester. Dieser Micellentyp bildet komplexe Strukturen, näherungsweise zu beschreiben als Micelle mit zwei Schalen. Der zweite Micellentyp enthält neben Wirkstoff mindestens einen Polyelektrolyten oder mindestens einen Glycerinester.

Dieser Micellentyp bildet vergleichsweise einfachere Strukturen, näherungsweise zu beschreiben als Micelle mit einer Schale. Es versteht sich, dass die Wirkstoffzusammensetzung verschiedene Micellen des ersten Typs und / oder verschiedene Micellen des zweiten Typs enthalten kann. die in Bsp. 1 dargestellte Zusammensetzung enthält Micellen (i), (ii) und (iv) des ersten Typs ("komplexe Micellen") und Micellen (iii) des zweiten Typs ("einfache Micellen"). Die Mizellen enthaltenden Wirkstoffzusammensetzungen können in Form von mizellaren Phasen oder mizellaren Dispersionen vorliegen. Mizellare Phasen und mizellare Dispersionen unterscheiden sich lediglich im Gehalt an Lösungsmittel, vorzugsweise Wasser oder eine wässrige Lösung.

Soweit sich aus dem Kontext nichts anderes ergibt, sollen die Begriffe Wirkstoff und Wirkstoffe, Glycerinester sowie Polyelektrolyt und Polyelektrolyte sowohl den Singular, wie auch den Plural (mindestens zwei) umfassen.

In Mizellen enthaltenden Dispersionen sind die Mizellen üblicherweise in einer wässrigen Phase dispergiert. Entsprechend kann die erfindungsgemässe Wirkstoffzusammensetzung als flüssige Formulierung mit einem vergleichsweise hohen Wasseranteil vorliegen. In dieser Ausgestaltung enthält die Wirkstoffzusammensetzung mindestens zwei voneinander verschiedene Typen von Mizellen, in einer wässrigen Phase. In dieser Ausgestaltung ist der Gehalt an Wasser in der Wirkstoffzusammensetzung typischerweise > 10 Gew.%.

Die erfindungsgemässe Wirkstoffzusammensetzung kann als feste Darreichungsform mit einem vergleichsweise niedrigen Wasseranteil vorliegen. In dieser Ausgestaltung ist der Gehalt an Wasser in der Wirkstoffzusammensetzung typischerweise < 10 Gew.%. Solche festen Darreichungsformen sind erhältlich, indem den flüssigen Formulierungen Wasser entzogen wird, bspw. durch Verdampfen. So Sind bspw. Dragees oder Kapseln erhältlich. Solche festen Darreichungsformen sind ferner erhältlich, indem den flüssigen Formulierungen Wasser entzogen wird, bspw. durch Gefriertrocknung. So Sind bspw. Pulver oder Tabletten erhältlich.

### Hilfsstoffe (Polyelektrolyte, Glycerinester)

Geeignete Fettsäureester sind niedermolekulare, amphiphile Ester mit ein bis drei Fettsäuren, je mit gleicher oder variabler C-Kettenlänge, die entweder gesättigt oder ungesättigt sein können und durch Glycerin oder allgemein eine C₃-Kette untereinander verbunden sind. Vorzugsweise handelt es sich bei den Fettsäuren um C₁₈-Ketten, wobei auch andere C-Kettenlängen wirksam und üblich sind.

Bevorzugte Fettsäureester sind Mono- und Diglyceride von Fettsäuren, d.h. Mono- bzw. Diester des Glycerins mit Fettsäuren. Bevorzugt sind Mono- und Diglyceride natürlich vorkommender Fettsäuren, wie Speisefettsäuren. Mono- und Diglyceride von Speisefettsäuren sind in der EU als Lebensmittelzusatzstoff unter der Nummer E 471 und weiter verestert mit verschiedenen Säuren als E 472a-f im Handel und sind ohne Höchstmengenbeschränkung (quantum satis) für Lebensmittel allgemein zugelassen. Bei der Veresterung zu E 472 werden je nach Buchstabe Essigsäure (E 472a), Milchsäure (E 472b), Citronensäure (E 472c), Weinsäure (E 472d), Mono- und Diacetylweinsäure (E 472e) oder gemischte Essig- und Weinsäure (E 472f) verwendet.

Glycerinester von Wurzelharzen sind in der EU ebenfalls als Lebensmittelzusatzstoffe unter der Nummer E 445 zugelassen. E 445 ist fettlöslich und hat eine höhere Dichte als Wasser. Hauptbestandteil der Wurzelharze sind Harzsäuren. Hergestellt wird E 445 durch Veresterung von Harzsäuren mit Glycerin. Als Harz wird das Harz der Sumpfkiefer (Pinus palustris) genutzt. Veresterung mit zusätzlichen Säuren, wie oben für die Fettsäureglyceride offenbart, sind weniger bevorzugt, aber ebenfalls umfasst.

Harzsäuren haben alicyclische Strukturen und weisen neben der Carboxygruppe oft noch weitere funktionelle Gruppen wie Hydroxy-, Keto- oder Aldehydgruppen auf. Es handelt sich im Wesentlichen um Diterpensäuren und Triterpensäuren.

Neben den Glycerinestern von Fettsäuren und Harzsäuren (einschliesslich weiterer Stoffe, die in natürlichen Gemischen vorkommen), oder anstelle solcher Glycerinester kann mindestens ein Polyelektrolyt vorhanden sein. Dieser mindestens eine Polyelektrolyt ist ausgewählt aus Oligosacchariden oder Polysacchariden mit Polyelektrolytcharakter, wie Gummi Arabicum, Cyclodextrine, Glykane, Glykogene, Glycolipide und Mischung derselben, insbesondere aber Gummi Arabicum und/oder Cyclodextrine. Für die Freisetzung im Dünndarm bevorzugte Polysaccharide sind Gummi Arabicum und Fraktionen von Gummi Arabicum.

Polyelektrolyte sind wasserlösliche Verbindungen mit großer Kettenlänge (Polymere), die anionische (Polysäuren)oder kationische (Polybasen) dissoziierbare Gruppen tragen.

Verbindungen mit Polyelektrolytcharakter gemäss der vorliegenden Erfindung sind Polyelektrolyte und Verbindungen mit einer Vielzahl, wie 15 und mehr, ionischen und/oder polaren Gruppen.

Ein bevorzugter Polyelektrolyt ist ein natürliches Polysaccharid mit linearen (1-3)-verknüpften-β(D)-Galactopyranosid-Gruppen in der Hauptkette, bei welchem zusätzlich - infolge Oxidation der Zucker-Seitenketten - Carboxygruppen vorhanden sind. Diese Carboxygruppen sind - soweit deprotoniert - als negative Ladungsträger des Polyelektrolyten entscheidend für die Wechselwirkung zwischen Polysacchariden und auch mit den Wirkstoffen, allfälligen Glycerinestern, allfälligen Hilfsstoffen und dem Lösemittel, insbesondere Wasser oder wässrige Lösungen. Die Carboxylatgruppen werden ausserdem durch die Mengen von ein- und zweiwertigen Kationen sowie den pH-Wert des umgebenden Lösemittels beeinflusst. Die Mengen an Polyelektrolyt, Glycerinester, Wirkstoff sowie Wasser und dessen pH-Wert, sind verantwortlich für die Ionenstärke, die wiederum die Mizellenbildung beeinflusst.

Der Polyelektrolyt ist vorzugsweise ein Polysaccharid mit variabler Kettenlänge und variabler Zahl an negativen Ladungen in den Seitenketten. Typische relative Molmassen der Polymere sind bis zu 1 Million , insbesondere 300 000 - 800 000.

Die Wechselwirkungen zwischen Polyelektrolyt, Glycerinester und Wirkstoff erfolgt wesentlich über Wasserstoffbrückenbildungen, Dipol-Kräfte und Van der Waals Wechselwirkungen, z.B. zwischen Wirkstoffen und Seitenketten des Polyelektrolyten.

Die wesentlichsten Bestandteile von Gummi Arabicum sind polydisperse Polyelektrolyt-Gemische von Copolymeren, insbesondere saure Erdalkali- und Alkalisalze der Arabinsäure (Polyarabinsäure), einem verzweigten, aus L-Arabinose, D-Galactose, L-Rhamnose und D-Glucuronsäure im Verhältnis 3:3:1:1 bestehenden Polysaccharid. Weitere Bestandteile von Gummi Arabicum umfassen Polygalaktose (poly-β-(1-6)-Galaktopyranose) mit vielverzweigten Zuckerketten, insbesondere 1-4-D-Galakturonsäure-Seitenketten sowie deren Alkalmetall- und Erdalkalimetallsalze.

Cyclodextrine gehören zu den cyclischen Oligosacchariden. Sie werden aufgrund der Anzahl ihrer Glucosemoleküle eingeteilt in alpha-Cyclodextrin (6 Glucosemoleküle), beta-Cyclodextrin (7 Glucosemoleküle), gamma-Cyclodextrin (8 Glucosemoleküle) und delta-Cyclodextrin (9 Glucosemoleküle). Derzeit bevorzugt ist alpha-Cyclodextrin.

Es können auch zusätzlich mehrwertige Alkohole, wie insbesondere Glycerin E422, als Hilfsstoff, z.B zur Einstellung der Viskosität beigegeben werden.

Die hier beschriebenen Mizellen sind aufgebaut aus variablen Anteilen der Hilfsstoffe Polyelektrolyte und/oder Glycerinestern. Insbesondere für gezielte Freisetzung ist ein Polyelektrolyt vorteilhaft. Da dieser bevorzugt ausgewählt ist aus natürlichen Polysacchariden mit Polyelektrolytcharakter, bewirkt dieser keinerlei Immunreaktionen. Diese vorteilhafte Eigenschaft ist bedingt durch die Nano-Skaligkeit sowie die spezifischen, natürlichen molekularen Bestandteile und - für das Naturprodukt Gummi Arabicum - die höchst relevanten Kohlenhydratstrukturen des Polyelektrolyten Arabinsäure als hauptsächlicher Komponente von Gummi Arabicum. Die ungefähre relative Molmasse der Arabinsäure beträgt Mᵣ = 3·10⁵ bis 1.1·10⁶.

Ohne sich an eine Theorie gebunden zu fühlen, wird davon ausgegangen dass die hier definierte Wirkstoffzusammensetzung auf der speziellen Struktur der Micellen beruht und so zu vorteilhaften pharmakologischen Effekten führt. Die folgenden Ausführungen zum Transport der Mizellen beruhen auf dem derzeitigen Wissensstand und sollen die Erfindung in keiner Art und Weise einschränken:
Der Transport der Mizellen in den Organismus kann oral über den Magen-Darmtrakt oder auch anderweitig erfolgen. Die orale Verabreichung ist die häufigste. Daneben können Mizellen aber auch topisch, intravenös oder - durch Inhalation eines Nebels aus Mikrotröpfchen, die durch ein Spraysystem erzeugt werden - über die Atemwege verabreicht werden.

Werden die Mizellen mit einer Fraktion des Gummi-Arabicum hergestellt, die einen hohen Anteil an D-Galakturonsäuren in den Seitenketten der Arabinsäure enthält, so liegen diese beim pH-Wert des Magens in mehrheitlich protonierter Form vor. Dies ergibt praktisch keine lokale, elektrischnegative Ladung bei pH=1 bis pH=2, was den sicheren und stabilen Einschluss der Wirkstoffe zusammen mit geeigneten Hilfsstoffen und deren sicheren Transport in den Darm begünstigt.

Ausserdem ist im Magen keine enzymatische Verdauung der Arabinsäure von Gummi Arabicum möglich, da im Magen und Dünndarm die spezifischen Enzyme zum substantiellen Abbau der mizellenbildenden Polyelektrolyte fehlen.

Der protonierte Polyelektrolyt, zusammen mit niedermolekularen (Wirk)stoffen nimmt im Magen eine sphärische Form an, die die (Wirk)stoffe einschliesst, was einen enzymatischen Angriff auf die Mizelle zusätzlich sterisch verhindert.

Mizellen, deren totale Ladung nach aussen gegen null tendieren, nehmen thermodynamisch begünstigt eine sphärische Form an. Auch Gegenionen, wie neutralisierende Alkalionen und praktisch neutralisierende Erdalkaliionen, spielen eine Rolle bei der Ladungsabschirmung.

Die Protonierung der Galakturonsäure, dem oxidierten Produkt der Galaktose, in den verzweigten Seitenketten der Arabinsäure, ist bei pH-Werten tiefer als 2, wie sie bekanntlich im Magen zu messen sind, praktisch vollständig. Der pKₐ-Wert der freien α-D-Galakturonsäure beträgt ca. 2.9. Die pKₐ-Werte der in Arabinsäure gebundenen, verschiedenen Galakturonsäure-Reste hängen von der lokalen Umgebung ab und sind stark von der lokalen Dielektrizitätskonstante, von der Möglichkeit zur Wasserstoffbrückenbildung, d.h. der lokalen sterischen Umgebung, und auch von der lokalen Ionenstärke beeinflusst.

In der wässrigen Phase des Zwölffingerdarms erhöht sich der pH-Wert durch alkalische Pankreassekrete von ca. pH = 8.2 und Bicarbonate schlagartig auf ca. pH = 6.6. Die dort vorhandene Gallensäure sowie die proteolytischen Enzyme Trypsin und Chymotrypsin führen zu keinem enzymatischen Abbau der Mizellen bzw. der darin eingeschlossenen Bestandteile.

Im Zwölffingerdarm werden die Fette der Nahrung, die in Form von Triglyzeriden vorliegen, durch Gallensäuren emulgiert. Die Triglyzeride werden enzymatisch durch Lipase in Monoglyzeride und Fettsäuren zerlegt, die Mizellen bilden. Diese Mizellen werden in die Darm-Epithelzellen des Dünndarms eingeschleust. In den Zellen werden sie im endoplasmatischen Retikulum der Darmepithelzellen zusammen mit Cholesterin und Lipoproteinen zu Chylomikronen zusammengebaut, welche mittels der Lymphgefässe schliesslich in die Blutbahn gelangen.

Unmittelbar beim Eintritt in den kurzen Zwölffingerdarm als oberster Teil des Dünndarms findet man die tiefsten pH-Werte. Beim Übergang vom kurzen Zwölffingerdarm in den nächsten Abschnitt des Dünndarms herrschen pH-Werte von ca. 6.6. Lokal herrschen in jedem Abschnitt des Dünndarms leicht unterschiedliche pH-Werte vor, d.h leicht saure Werte um ca. pH = 6.5.

Beim Eintritt in den oberen Dickdarmabschnitt erniedrigt sich der pH-Wert auf ca. 5.5. Diese pH-Senkung wird durch kurzkettige Fettsäuren der sie produzierenden Darmbakterien verursacht. Diese sauren, bakteriellen Produkte werden über die Länge des Dickdarms resorbiert wodurch ein leichter pH-Anstieg im Dickdarm bis zu dessen Darmende erfolgt.

Die Polyelektrolyt-Mizellen geben Ihre Wirkstoffe vor allem im Dünndarm frei oder werden integral in Zellen eingeschleust, wo sie in ihre Bestandteile zerlegt werden. Die im Dünndarm mit der pH-Erhöhung verbundene Deprotonierung bewirkt eine teilweise Entfaltung der negativ geladenen Arabinsäure-Seitenketten durch Ladungs- Abstossungskräfte, wodurch die leicht gebundenen Stoffe aus den Mizellen teilweise herausgelöst werden.

Je nach Typ der Mizelle, des Polyelektrolyten und/oder des Glycerinesters, allfälliger Hilfsstoffe und der aktiven Inhaltsstoffe (Wirkstoffe) erfolgt der Transfer der Mizelle in das Darmepithel über die gesamte Länge des Dünndarms und erlaubt den effizienten Transport der niedermolekularen, aktiven Stoffe in den Blutkreislauf.

Parallel dazu oder als Alternative können die mizellierten Wirkstoffe über die Darmepithelzellen in die Blutbahn eingeschleust werden. Es wird angenommen, dass diese durch das Andocken der vielfältigen Zuckerbestandteile der Mizellen an die ebenso vielfältigen Kohlenhydratfunktionen von membrangebundenen Proteinen ermöglicht wird. Dies wird als erster Schritt zur Überwindung der Zellmembran-Barriere und des Transports der Wirkstoffe der Mizellen ins Zellplasma angesehen.

Die Schleimhäute zwischen dem Darmlumen und den Darmepithelzellen schützen die Zellen vor der enzymatischen Verdauung. Die Schleimhäute müssen von den Mizellen mittels Diffusion durchwandert werden.

Es wird angenommen, dass die lateralen, molekularen Kräfte, welche durch die Bindung der Mizellen an die Kohlenhydratfunktionsstellen entstehen, auf die anfänglich noch intakte Zellmembran wirken, diese dann lokal schwächen bzw. öffnen, und dadurch den Eintritt der Wirkstoffe in die Zelle bzw. die Einschleusung der Mizellen in die Endothelzellen erlauben, wo die Mizellen Ihren Inhalt freigeben. Während der grösste Teil der Kohlenhydrate im Zwölffingerdarm und dem anschliessenden Leerdarm aufgenommen wird, können die dafür verantwortlichen Enzyme die mizellenbildende Arabinsäure nicht hydrolytisch zerlegen, wie das für gewisse andere langkettigen Kohlenhydrate der Fall ist. Gummi Arabicum aus Mizellen, deren Wirkstoff im Dünndarm freigesetzt und resorbiert wurde, kann nur im Dickdarm mit Hilfe von Bakterien abgebaut werden, da die von menschlichen Zellen zur Verfügung gestellten Enzyme dieses Polysaccharid nicht verdauen können. Gummi Arabicum gilt deshalb als löslicher Ballaststoff, der in grösseren Mengen den Wassergehalt des unverdauten Inhalts im Dickdarm erhöht.

Die Bildung stabiler Mizellen wird durch geeignete Wahl der Glycerinester und/oder des Polyelektrolyten verbessert. Die Wahl des Glycerinesters und insbesondere auch des Polyelektrolyten hängt von den jeweiligen Wirkstoffen und den Eigenschaften der Fraktion von Gummi Arabicum, sowie dem Lösemittel ab.

Wirkstoffe mit Dipolmoment oder Ladung lassen sich besser in Zusammensetzungen einbringen, die reich sind an Polyelektrolyt, insbesondere in Zusammensetzungen, die > 50 % Polyelektrolyt bezogen auf die Gesamtmenge an Polyelektrolyt und Glycerinester enthalten, vorzugsweise > 75 % Polyelektroly enthalten.

### pH Wert

Durch Einstellung des pH-Werts kann bei den bevorzugten, geladenen (carbonylatgruppenhaltigen) Polysacchariden zudem die einarbeitbare Menge an im deprotonierten Zustand ionischen Wirkstoffen optimiert werden, d.h. Wirkstoff und Polysaccharid liegen vorzugsweise beide mehrheitlich protoniert oder deprotoniert vor. Beispielsweise Ascorbinsäure (pKa = 4,25) lässt sich besser bei tiefem pH-Wert in eine Zusammensetzung mit hohem Gehalt an Gummi Arabicum oder aus Gummi Arabicum bestehend (pKa-Wert der Arabinsäure = 4.4 bis 4.93) einarbeiten.

Für Polyelektrolyte enthaltende mizellare Zusammensetzungen ist der pH vorzugsweise kleiner als der pKₐ der Seitenketten, wie pH < 4, insbesondere < 3.5, speziell bevorzugt ca. 3.2. Bei pH-Werten von 3.2 sind die Polymer-Seitenketten mehrheitlich ungeladen und die elektrostatische Coulomb-Wechselwirkung innerhalb und zwischen den Polyelektrolytmolekülen sinkt zunehmend ab. Bei steigenden pH-Werten erfolgt Deprotonierung der Carbonsäuregruppen in den Seitenketten, was die Freisetzung der Wirkstoffe erleichtert oder erst ermöglicht ("Entknäuelung" bzw "defolding" der sphärischen Partikel).

Die Einstellung des pH-Wertes erfolgt vorzugsweise mittels natürlicher Säuren, wie Zitronensäure, Äpfelsäure etc.

### Weitere Komponenten und Parameter

Ein geringer Gehalt an Proteinen und Glykoproteinen kann die Mizellenbildung beeinflussen. Bevorzugt sind die in natürlichen Ausgangsstoffen, wie Polysacchariden, als Begleitstoffe enthaltenen Proteine und Glycoproteine. So verbessern die in Gummi Arabicum enthaltenen geringen Mengen an pflanzlichen Proteinen die Mizellenbildung von unpolaren Stoffen oder ermöglichen diese erst. Der massenmässige Protein-Anteil des Polysaccharids beträgt im Allgemeinen weniger als 5%, üblicherweise aber mindestens 0.5 %, vorzugsweise 1.5 -2.5 %.

Die Mizellenbildung kann folglich von den Wirkstoffen und/oder vom (üblicherweise geringen) Gehalt an pflanzlichen Proteinen in den Akazienarten abhängen, aus denen der Gummi Arabicum gewonnen wurde. Die üblicherweise vorhandenen ein bis zwei Massenprozente an solchen Proteinen hat sich vor allem bei der Stabilisierung von Emulsionen fettlöslicher Wirkstoffe durch Mizellierung als sehr vorteilhaft erwiesen.

Für den Einschluss verschiedener Wirkstoffe unterschiedlicher Polarität und molekularer Struktur in die Mizellen sind die thermodynamischen Aspekte der Wechselwirkung der einzelnen an der Mizellenbildung beteiligten Stoffe relevant, insbesondere des Lösemittels Wasser, des Polyelektrolyten und/oder des Glycerinesters.

Die Summe der Änderungen von energetischen und entropischen Anteilen definieren die Differenz der freien Energie und damit das Ausmass der Mizellenbildung, sowie die thermodynamische Stabilität der Mizellen. Die freie Energieänderung ΔG muss dabei negativ sein (ΔG = ΔH - TΔS).

Die energetischen Anteile (ΔH) umfassen die Wechselwirkungen der hydratisierten Protonen bzw. der Alkali- oder Erdalkali-Ionen mit den basischen Carboxylatresten der Arabinsäure und die Wechselwirkungen der polaren Teile und Teilbereiche der Polyelektrolyte, der Glycerinester und gegebenenfalls der Hilfsstoffe mit den Wirkstoffen. Hilfsstoffe sind z.B. die in natürlichem Gummi Arabicum enthaltenen Pflanzenproteine, können aber auch andere Stoffe mit emulgierenden und/oder dispergierenden Eigenschaften sein.

Apolare Teile der Glycerinester und/oder der Hilfsstoffe wechselwirken bevorzugt mit apolaren Wirkstoffen, da die Arabinsäure - selbst im ungeladenen Zustand - keine apolaren Molekülfragmente besitzt.

Die entropischen Anteile (ΔS) sind in erster Linie von der Zahl freier Moleküle abhängig. Die freien Moleküle schliessen auch die Anteile der am Geschehen beteiligten Lösemittelmoleküle, wie Wasser, ein, sowie die Polyelektrolyte und/oder die Glycerinester, gegebenenfalls Hilfsstoffe und die Wirkstoffe.

Zusätzlich können entropische Beiträge aus der Konformationsänderung des Polyelektrolyten wichtig sein. Es wird angenommen, dass das elektrostatische Feld in der Nähe insbesondere der negativ geladenen Carboxylatreste von gebundener Glukuronsäure, zusätzlich zur Polarisation und Wasserstoffbrückenbildung, eine lokale Ordnung der polaren Wassermoleküle bewirkt, wodurch die lokale Dielektrizitätskonstante gegenüber freiem Wasser lokal stark absinkt. In der Nähe dieser Carboxylatreste oder allgemein von Ladungsresten des Polyelektrolyten bewegen sich die verschiedenen hydratisierten Kationen und polarisierbaren Moleküle unter dem Einfluss des lokalen elektrische Feldes des geladenen Polyelektrolyten.

Die pKa-Werte der Polyelektrolyte sind, zusammen mit den gewählten pH-Bedingungen und den Kationen in der flüssigen Phase, wesentlich für den globalen Ladungszustand der molekularen Aggregate.

In freier Lösung ohne Ionen bestimmen im Wesentlichen die Grösse der Moleküle, die Temperatur, die Natur des Lösemittels und die Dipol-Kräfte mit ihrer polarisierenden Wirkung auf die Nachbarteilchen die Diffusion aller Mischungspartner.

Für Polyelektrolyte wird angenommen, dass deren Konformationsänderung vom quasi linearen Zustand in den quasi sphärischen Zustand, bzw. die Aggregatbildung zur Formung von Mizellen, einhergeht mit der zuerst zu erfolgenden partiellen Neutralisation oder Abschirmung der negativen Ladung der Carboxylatgruppen in den Seitenketten (Glukuronsäure) durch Protonen oder Kationen und der darauf folgenden Bildung von intramolekularen Wasserstoffbrücken sowie allenfalls Wasserstoffbrückenbildung mit den Wirkstoffen, und/oder mit den Glycerinestern. Die Konformationsänderung von quasi linear zu quasi sphärisch bewirkt auch das partielle Einfrieren von Freiheitsgraden der Rotation in der Haupt- und teilweise in den vielen verzweigten Seitenketten des Polyelektrolyten. Diese Prozesse sind mit einer negativen Entropieänderung verbunden, da die Beweglichkeit der Teilchen eingeschränkt wird. Diese negative Entropieänderung wird durch die vorwiegend negativen Enthalpieänderungen bei der Bildung von neuen Wasserstoffbrücken kompensiert, so dass die gesamte freie Energie aller Prozesse zusammen negativ wird. Dies gilt insbesondere, falls Druck und Temperatur bei der Bildung konstant gehalten werden.

Die Kinetik der Protonierung von Carboxylaten ist schnell und diffusionskontrolliert. Auch Konformationsänderungen der Seitenketten sind schnelle Prozesse, wohingegen die Konformationsänderung der langen Hauptkette von Arabinsäure mehr Zeit beansprucht.

Falls mehrere Polyelektrolyte, wie Arabinsäureketten oder Moleküle mit vielen polaren Gruppen an der Bildung einer Mizelle beteiligt sind, steigen der Umfang und das Ausmass möglicher Wechselwirkungen unter allen beteiligten Stoffen.

Die Einteilung der Polarität erfolgt nach der Definition der Differenz der Elektronegativität der an der chemischen Bindung beteiligten Atome gemäss Pauling.

**Tabelle 1: (ΔEN = Differenz der Elektronegativität)**

| **Differenz der EN-Werte (Δ*EN*) für die Einteilung der Polarität einer Bindung** | | |
|---|---|---|
| **Δ*EN*** | **Bindungsart** | **Charakter der Bindung** |
| 0 | unpolare Bindung | Elektronenpaare werden von beiden Atomkernen exakt gleich stark beansprucht. |
| | | Es entsteht kein partielles Ladungsungleichgewicht. |
| | | (kein Dipol) |
| 0.1-0.4 | schwach polare Bindung | Ein Atomkern beansprucht das Elektronenpaar leicht stärker als der andere. |
| | | Leicht partielles Ladungsungleichgewicht (symbolisiert mit δ+. δ-) |
| | | (schwacher Dipol) |
| 0.4-1.7 | stark polare Bindung | Ein Atomkern beansprucht das Elektronenpaar deutlich stärker als der andere. |
| | | Damit ist die partielle Ladungsverteilung stark asymmetrisch. |
| | | (starker Dipol) |
| > 1.7 | Ionenbindung | Es sind keine gemeinsamen Elektronenpaare vorhanden. |
| | | Ein Atomkern beansprucht beide Elektronen (Ionenbildung; vollständige Ladungstrennung) |

Die Wechselwirkung in Polyelektrolyten, wie Arabinsäure, hat eine grosse Reichweite und kommt dadurch zustande, dass jeweils positiv geladene Ionen zwischen zwei negativ geladenen Teilchen liegen und so die Mizellenbildung oder die Clusterbildung der Polyelektrolyte induzieren. Ähnlich verhält es sich bei Molekülen mit vielen polaren Gruppen, wie den Cyclodextrinen. Sobald eine genügende Zahl von gegenpoligen Ladungsträgern vorhanden ist, erfolgt die Mizellenbildung schlagartig. Involviert in die Mizellenbildung sind mehrfach geladene Gegenionen, Dipol-Dipol-Kräfte, Quadrupol-Wechselwirkungen mit dem Polyelektrolyten, Wasserstoffbrückenbildung und Kationen-π-Wechselwirkungen von Doppelbindungen. Das ganze Geschehen kann als assoziatives Verhalten von Polyelektrolytlösungen bezeichnet werden.

Die clusterartige Mizellenbildung hinterlässt quasi leere Räume im Lösemittel, die lediglich mit Molekülen des Lösemittels besetzt sind, aber keine Ionen enthalten, da sich diese bevorzugt in der Nähe der gegenpoligen Ladungen von Teilen des Polyelektrolyten oder von Dipolen aufhalten.

Während die obige Beschreibung wesentliche Aspekte mehrheitlich am Beispiel der Arabinsäure aufzeigt, gelten diese auch für andere Polyelektrolyte, insbesondere Cyclodextrine, Glykane, Glykogene, Glycolipide und Mischung derselben. Speziell bevorzugt sind aber Gummi Arabicum und/oder Cyclodextrine.

Das Gewichts-Verhältnis von Glycerinester zu Polyelektrolyt kann in grossen Bereichen variieren, je nachdem, welche Eigenschaften bevorzugt werden sollen. In der Regel wird für einen ionischen/dipolaren Wirkstoff im Vergleich zu einem apolaren/ungeladenen Wirkstoff mehr Polysaccharid eingesetzt.

Das Verhältnis der Hilfssoffe, Glycerinester : Polysaccharid, genormt auf eine Summe von 100%, kann von 1% : 99% bis 99% : 1% variieren, vorzugsweise von 10% : 90% bis 90% : 10%, stärker bevorzugt von 20% : 80% bis 80% : 20%, insbesondere 25 ± 2 % : 75 ± 2 % oder 50 ± 2 % : 50 ± 2 % oder 75 ± 2 % : 25 ± 2 %, wie 25 % : 75 % oder 50 % : 50% oder 75% : 25 %.

### Wirkstoffe

Die erfindungsgemässen Mizellen enthaltenden Wirkstoffzusammensetzungen können eine grosse Vielzahl an unterschiedlichen Wirkstoffen schützen und an den gewünschten Aufnahme- bzw. Bestimmungsort transportieren. Als Wirkstoffe können praktisch beliebige Wirkstoffe eingesetzt werden. Aufgrund der Verträglichkeit sind aber Wirkstoffe natürlichen Ursprungs oder naturidentische, synthetische Substanzen, sowie Substanzen deren biologisch-biochemisches Wirkprinzip mit dem natürlicher Substanzen identisch ist, bevorzugt.

Die Wirkstoffe können ionisch, polar, wenig polar oder apolar sein und sie können Salze, kleine Moleküle, monomere Naturstoffe, Oligomere, kleine Peptide, Proteine, Oligonukleotide oder Polynukleotide sein.

Eine Liste möglicher Wirkstoffe, die einzeln oder als Mischung von Substanzen innerhalb einer Stoffklasse (unten fett gedruckt) oder als Mischung von Stoffen aus zwei oder mehreren dieser Stoffklassen eingesetzt werden können, ist nachfolgend angefügt:
**Aminosäuren,** wie *Essentielle Aminosäuren, z.B.* Tryptophan (Hydroxytryptophan), L-Phenylalanin, L-Methionin, L-Lysin und/oder *bedingt essentielle Aminosäuren, z.B.* Glutamin, L-Glutaminsäure, L-Cystein, und/oder L-Ornithin und/oder N-Acetylcystein;
**Natürliche Fettsäuren,** wie essentielle Fettsäuren, z.B. Linolsäure, Linolensäure, Omega-3-Fettsäuren, und/oder Gemische von natürlichen Fettsäuren: Maiskeimöl, Weizenkeimöl, und/oder Phospholipide und/oder Phosphatidylserin;
**Säuren/Basen,** d.h. protoniert und/oder deprotonierte bekannte Wirkstoffe mit pharmakologischer Wirkung, jedoch anders als natürliche Fettsäuren, Aminosäuren oder Hilfsstoffe zur pH Einstellung;
**Peptide und Oligopeptide,** wie Peptide und Oligopeptide humanen oder pflanzlichen oder tierischen Ursprungs;
**Proteine,** wie Insulin, Transferrin, Coeruloplasmin, Hämopexin, Globuline, Albumine, Antikörper, z.B. monoklonale Antikörper (Biologika);
**DNA, RNA,** wie Oligonukleotide und/oder Polynukleotide humanen, tierischen, bakteriellen oder viralen Ursprungs;
**Essentielle ionische Stoffe, wie** Chlorid, Sulfat, Phosphat, Iodid, Carbonat, Hydrogencarbonat, Borat, (jeweils mit einem physiologisch verträglichen Kation, bsp. die korrespondierenden Na-Salze), Borsäure;
**Antioxdidantien,** wie Vitamin C und dessen Salze, z.B. Ascorbylpalmitat, Ascorbylstearat, Vitamin E und dessen Salze, z.B. Tocopherole, Tocopherolacetat, a-(R+)-Lipon-säure und dessen Racemat, Glutathion, Thiomilchsäure, L-Cystein, Carotinoide, z.B. Lycopin, beta-Carotin, Lutein;
**Redoxaktive Substanzen,** wie Reduktionsmittel/Oxidationsmittel;
**Naturstoffe aus Pflanzen:** Polyphenole (Resveratrol, Flavonoide) Curcumine, Bromelain, Papain, Olibanum, Artemisinin,
**Naturstoffe aus anderen Lebewesen, Mikroben, Algen, Pilzen und daraus** hergestellte Mischungen/Extrakte, wie Astaxanthin, Lutein, Zeaxanthin, Cholin, Lezitin, Canthaxanthin Arbustin;
**Vitamine und modifizierte Vitamine,** wie Vitamin A (Retinol), Provitamin A, Vitamin-B-Komplex und dessen Derivate, z.B. B1 (Thiamin, B2 (Riboflavin), B3 (Niacin, Nicotinamid, Nicotinsäure), B4 (Adenin, Cholin), B5 (Pantothensäure), B6 (Pyridoxin), B7 (Biotin), B8 (Adenosinphosphat), B9 (Folsäure), B11 (Folsäure), B12 (Cobalamin, Methylcobalamin, Cyanocobalamin, Cobalamin, B15 (Pangamsäure) B17 (Amygdalin), Vitamin C (Ascorbinsäure) und dessen Salze, z.B. mit Na, Mg, Ca, Vitamin D, insbesondere D3 (Cholecalciferol), Vitamin E (Tocopherol), Vitamine K, K1, K2, K3 Vitamin-P-Gruppe (Bioflavonoide), para-Aminobenzoesäure (PABA) ;
**Essentielle Mineralstoffe,** wie Natrium, Kalium, Magnesium, Calzium, Selen, Selenoproteine, Selenit, Selenat, Eisen, Zink, Kupfer, Mangan, Kobalt, Zinn, Chrom, Nickel, Vanadium, Molybdän, Silizium, Iod, etc.;
**Naturstoffe, wie vitaminähnliche Naturstoffe, polymere Naturstoffe, oligomere Naturstoffe, und Monomere von Naturstoffen,** z.B. alle Zucker, Inulin, beta-Glucan, Coenzym Q10, L-Carnitin, Carnosin, Kreatin, Taurin, Orotsäure, Cholin, Inosit;
**Enzyme,** wie Amylasen, Pepsin, Trypsin, Chymotrypsin, Kathepsin, Chymosin, Di-Saccharidasen (Saccharase, Malatase, Laktase) Lipasen, Proteasen, Peptidasen, Elastasen, Hydrolasen, Isomerasen, ¨Ligasen, Lyasen, Oxidoreduktasen, Transferasen, Superoxiddismutase, Glutathionperoxidase, Katalase;
**Hormone,** wie Melatonin;
**Neurotransmitter,** wie Serotonin; sowie
mit radioaktiven Isotopen markierte Stoffe der Substanzen aus den obigen Stoffklassen und/oder mit stabilen Isotopen markierte Stoffe dieser Substanzen.

In einer Ausgestaltung weist die erfindungsgemässe Wirkstoffzusammensetzung verschiedene Wirkstoffe, wie verschiedene Naturstoffe, auf. In dieser Ausgestaltung weist der erste Typ von Micellen einen ersten Wirkstoff auf und der zweite Typ von Micellen einen zweiten Wirkstoff. Beispielhaft sei erwähnt die Kombination aus mindestens zwei, bevorzugt allen vier, der folgenden Wirkstoffe Curcumin , Olibanum , Artemisinin , Vitamin C. Wobei die Anwesenheit von Vitamin C - neben seiner positiven pharmazeutischen Wirkung - aufgrund seiner Wirkung als Anti-Oxidans auch zu Stabilisierungszwecken bevorzugt ist.

Das Gewichts-Verhältnis von Wirkstoffen zu Polysaccharid und/oder Glycerinester kann in einem weiten Bereich variieren. In einer Ausgestaltung ist die Beladung der Mizellen mit einem oder mehreren Wirkstoffen zwischen 0.1% (m/m) bis 90% (m/m) In einer weiteren Ausgestaltung ist das Verhältnis im Bereich von 4 : 1 bis 1 : 4, vorzugsweise von 3 : 1 bis 1 : 3, wie 2 : 1 bis 1 : 2 (m/m).

### Herstellung

Die Erfindung betrifft auch verschiedene Verfahren zum Herstellen von Wirkstoffzusammensetzungen enthaltend Micellen.

Ein Verfahren zur Herstellung einer mizellaren Phase oder einer Mizellen enthaltenden Dispersion welches die folgenden Schritte umfasst:
- Mischen aller Komponenten und Rühren derselben bei einer ersten Temperatur, bei der zumindest die Polyelektrolyte und die Glycerinester flüssig vorliegen, während einer vorbestimmbaren Zeit,
- Zugabe von Wasser, wobei das Wasser auf eine zweite Temperatur aufgeheizt ist, die der ersten Temperatur minus maximal 15°C entspricht und
- Rühren bei der zweiten Temperatur während eines vorbestimmbaren Zeitraums zur Bildung der mizellaren Phase oder der Mizellen enthaltenden Dispersion.

Ein bevorzugtes Verfahren zur Herstellung von Wirkstoff und Polyelektrolyt enthaltenden Mizellen umfasst:
- einen **ersten Schritt,** in dem durch Mischen (insbesondere unter Rühren) mindestens ein Polysaccharid und gegebenenfalls mindestens ein Glycerinester getrocknet werden und das mindestens eine Polysaccharid anschliessend bei einer ersten Temperatur geschmolzen oder durch Zugabe mindestens eines Glycerinesters gelöst wird,
- einen **zweiten Schritt** in dem durch Mischen (insbesondere unter Rühren) der Wirkstoff bei der ersten Temperatur zugemischt und die Mizellenbildung vorbereitet wird, indem gewartet wird, bis die durch Zugabe des Wirkstoffs trüb gewordene Zusammensetzung wieder klar wird,
- einen **dritten Schritt,** in dem zur klaren Zusammensetzung aus dem zweiten Schritt durch Mischen (insbesondere unter Rühren) Lösungsmittel, insbesondere Wasser oder eine wässrige Lösung, vorzugsweise vorbestimmten pH-Werts, zugegeben, wird, worauf sich selbstregulierend sphärische Formen ("Mizellen"), bilden.

Ein für dieses Verfahren bevorzugter Polyelektrolyt ist ein Polysaccharid mit Carboxygruppen in Seitenketten.

Auch in diesem Verfahren hat das im dritten Schritt zugegebene Wasser oder die wässrige Lösung bevorzugt eine zweite Temperatur, die der ersten Temperatur, d.h. der Temperatur der Zusammensetzung aus dem zweiten Schritt, minus maximal 15°C entspricht.

Ob eine mizellare Phase oder eine Mizellen enthaltende Dispersion erhalten wird, hängt lediglich von der Menge Wasser ab, das zugegeben wird. Für die Herstellung einer mizellaren Phase wird nur eine gerade zur Ausbildung der Mizellen benötigte Menge Wasser zugegeben, wofür üblicherweise eine Menge von ca. 0.1 Gew.-% Wasser bezogen auf das Gewicht der Wirkstoffe und des Polyelektrolyten und/oder des Glycerinesters ausreicht. Das Wasser wird zudem infolge der erhöhten Temperatur nach Bildung der Mizellen zum grössten Teil verdampft, so dass eine nahezu trockene mizellare Phase vorliegt. Diese kann durch moderate Temperaurerhöhung (auf ca. 40-50°C, wie 45°C) fliessfähig gemacht werden, so dass sie z.B. in Kapseln abgefüllt werden kann.

Für die Herstellung einer Mizellen enthaltenden Dispersion hat sich eine Wassermenge von ca. 20 bis ca. 50 Gew.-% bezogen auf das Gewicht der Mizellen in der Mizellen enthaltenden Dispersion, insbesondere 30 bis 40 Gew.-%, als bevorzugt herausgestellt.

Das Verfahren zur Herstellung einer mizellaren Phase oder einer Mizellen enthaltenden Dispersion kann bei Umgebungsdruck durchgeführt werden. Es hat sich aber als vorteilhaft herausgestellt, wenn die Herstellung in einem geschlossenen Behälter erfolgt und ein geringer Überdruck zugelassen wird. Ein solcher Überdruck, im allg. von < 5 bar, insbesondere < 1 bar, reduziert die Schaumbildung, verkürzt die Herstellungsdauer und verbessert oft auch die Homogenität.

Die erfindungsgemässe Zusammensetzung erfolgt durch einfaches Vermischen von zwei oder mehr mizellaren Phasen und/oder mizellaren Dispersionen, wobei die Ionenstärke, pH - Wert und Micellengrösse vorteilhaft von gleicher Grössenordnung (d.h. jeweils +/- 10 des jeweiligen Parameters) sind.

Die Trocknung im ersten Schritt erfolgt üblicherweise durch Erhitzen, gegebenenfalls und bevorzugt unter Vakuum. Die Polyelektrolyt-Glycerinester-Zusammensetzung im ersten Schritt ist so zu wählen, dass für einen ionischen/dipolaren Wirkstoff im Vergleich zu einem apolaren/ ungeladenen Wirkstoff mehr Polyelektrolyt eingesetzt wird. Vorzugsweise sind die Temperatur und der Gasdruck so zu wählen, dass die Polyelektrolyte und Glycerinester vollkommen verflüssigt und auf konstanter Temperatur sind. Im zweiten Schritt wird die Zusammensetzung nach Zugabe des Wirkstoffs vorzugsweise entgast.

Im dritten Schritt zugegebenes Wasser oder zugegebene wässrige Lösung wird vorzugsweise auf einen zuvor bestimmten pH-Wert eingestellt. Vorzugsweise wird das Wasser oder die wässrige Lösung auf eine zweite Temperatur eingestellt, die der ersten Temperatur, d.h. der Temperatur der Zusammensetzung aus dem zweiten Schritt, minus maximal 15°C entspricht.

Für Polysaccharide enthaltende mizellare Zusammensetzungen ist der pH vorzugsweise kleiner als der pKₐ der Seitenketten, wie pH < 4, insbesondere <3.5, speziell bevorzugt ca. 3.2. Bei pH-Werten von 3.2 sind die Polymer-Seitenketten mehrheitlich ungeladen und die elektrostatische Coulomb-Wechselwirkung innerhalb und zwischen den Polyelektrolytmolekülen sinkt zunehmend ab. Bei steigenden pH-Werten erfolgt Deprotonierung der Carbonsäuregruppen in den Seitenketten, was die Freisetzung der Wirkstoffe erleichtert oder erst ermöglicht.

Es wird angenommen, dass die Mizellenbildung bei Zugabe des Lösungsmittels erfolgt, um die Zusammensetzung energetisch zu minimieren und dass die Polyelektrolyte und gegebenenfalls die Glycerinester die Wirkstoffe in einer geordneten Hülle thermodynamisch stabilisieren und gegen das sie umgebende Lösemittel abschliessen.

In einer bevorzugten Ausgestaltung des Herstellungsverfahrens wird die Temperatur in allen Verfahrensschritten in etwa konstant gehalten, also +/- 5°C oder weniger.

Falls der Wirkstoff oxidationsempfindlich ist, kann in der 2. und 3. Stufe unter Inertgas gearbeitet werden.

Der Gasdruck über dem Reaktionsgemisch kann vorzugsweise zwischen 0.5 bar bis 2 bar variieren. Typisch ist ein GasDruck von 0.8 bis 1 bar. Die Temperatur des Reaktionsgemisches variiert zwischen 0°C bis 100°C, typisch sind 30°C bis 95°C je nach Wirkstoff. Die Art des Gases oder des Gasgemisches wird - falls verwendet - den an der Mizellenbildung beteiligten Stoffe angepasst und übt teils eine Schutzwirkung, insbesondere auf die Wirkstoffe aus. Übliche Gase sind Inertgase, wie N2 und Ar, aber auch CO2.

Für die genannten Verfahren gilt:
- Eine mizellare Phase kann hergestellt werden, indem eine Menge Wasser zugegeben wird, die gerade ausreichend ist, für die Mizellenbildung, insbesondere ca. 0.1 Gew.-%.
- Eine Mizellen enthaltenden Dispersion kann hergestellt werden, indem eine Wassermenge von ca. 20 bis ca. 50 Gew.-% bezogen auf das Gewicht der Mizellen, insbesondere 30 bis 40 Gew.-%, zugegeben wird.
- Die Zeit, die für das Mischen und Rühren aller Komponenten, bzw. in den Schritten 1 und 2, benötigt wird, kann festgelegt werden, indem die Qualität der Mizellen nach unterschiedlich langen Zeiten bestimmt und die Zeiten gegebenenfalls extrapoliert werden.
- Die für die Mizellenbildung benötigte Zeit kann visuell bestimmt werden.
- Die erfindungsgemässe Zusammensetzung kann durch einfaches Vermischen von zwei oder mehr mizellaren Phasen und/oder mizellaren Dispersionen erfolgen, wobei auf auf die vorstehend genannten Parameter zu achten ist.

### Beispiele

Die Erfindung wird nun anhand einiger **Beispiele** näher erläutert:

### Beispiel 1: (Wirkstoffkombination + Solubilisierungs- bzw. Mizellierungshilfstoffe):

Es wurden Einzel-Zusammensetzung hergestellt aus
(i) 60 g Curcumin, 60 g Gummi Arabicum und 40g Glycerinester von Wurzelharzen (E 445)
(ii) 30 g Olibanum, 30 g Gummi Arabicum und 10g Glycerinester von Wurzelharzen (E 445)
(iii) 100 g Vitamin C und 100 g Gummi Arabicum
(iv) 20 g Artemisinin, 100 g Gummi Arabicum und 50g Glycerinester von Wurzelharzen (E 445)

Die Herstellung der Einzel-Zusammensetzungen erfolgte, indem alle Komponenten vorgelegt und unter Rühren während 1h bei 105°C temperiert wurden. Anschliessend wurde zu jeder Einzel-Zusammensetzung eine für die Mizellenbildung gerade ausreichende Menge Wasser von 95°C zugegeben, die Einzel-Zusammensetzungen anschliessend zusammengegeben und gemischt. Dann wurde mit Wasser von 95°C auf einen Wirkstoffgehalt von 21 % verdünnt. Die Reaktionsmischung wurde während 5 min bei 92°C gemischt, bei dieser Temperatur entgast und, zur Abtrennung grösserer nicht mizellarer Strukturen, filtriert. Es wurden 1000g einer, stabilen Dispersion erhalten.

Diese konzentrierte Zusammensetzung mit 21 % Wirkstoffkombination wurde anschliessend auf 1 % Wirkstoffkombination, 2.5% Wirkstoffkombination, 5% Wirkstoffkombination und 10 % Wirkstoffkombination verdünnt. Die visuelle Beurteilung (Zusammenfassung der Beurteilung mehrerer Proben) ist in Tabelle 2 wiedergegeben.

**Tabelle 2:**

| **Verdünnung** | **Beurteilung** |
|---|---|
| 1% | klar / transparent schnell lösend; 5 FTU |
| 2,5% | klar / transparent - opakisierend schnell lösend; >5 FTU - 10 FTU |
| 5% (bevorzugt) | klar / transparent - opakisierend schnell lösend; <2 FTU - 5 FTU |
| 10% | klar-trüb / opakisierend schnell lösend; <5 FTU - 5 FTU |

| | |
|---|---|
| FTU: Formacine turbidity units, gemessen bei 860 nm, vgl ISO 7027. | |

**Curcumin** oder (1E,6E)-1,7-Bis(4-hydroxy-3-methoxyphenyl)-hepta-1,6-dien-3,5-dion ist der Hauptbestandteil von Kurkuma/Turmeric Root Extract, das neben Curcumin auch Derivate desselben (Curcuminoide) enthält, typischerweise in der Zusammensetzung: Curcumin (60 %), Demethoxycurcumin (25 %) und Bisdemethoxycurcumin (15 %). Die Curcuminoide können zusammen mit Sesquiterpenen vorliegen, wie Turmeron (bis 30 %), ar-Turmeron (bis 25 %), Atlanton und Zingiberen (bis 25 %) und Monoterpenen (Cymen, 1,8-Cineol, Phellandren, Sabinen, Borneol.

**Olibanum** (Weihrauch) ist typischerweise zusammengesetzt aus:
- Ca. 15 bis 16 % Harzsäuren, wie Boswelliasäuren, Lupansäuren und Tirucallensäuren, mindestens je 1 % 3-O-Acetyl-11-keto-β-boswelliasäure (AKBA) und 11-Keto-β-boswelliasäure (KBA)
- Ca. 5-9 % ätherischen Ölen, wie alpha-Thujen, beto-Myrcen, p-Cymol und Methyleugenolund
- Bis 20 % Schleimstoffe.

Eine Fraktion ohne Schleimstoffe ist bevorzugt.
**Ascorbinäure und deren Derivate/Vorläufer (Vitamin C),** L-(+)-Ascorbinsäure, (5R)-5-[(1S)-1,2-Dihydroxyethyl], 3,4-dihydroxy-5-hydrofuran-2-on (IUPAC), 3-Oxo-L-gulonsäure-γ-lacton
**Artemisinin** (3R,5aS,6R,8aS,9R,125,12aR)-Octahydro-3,6,9-trimethyl-3,12-epoxy-12H-pyrano[4,3-j]-1,2-benzodioxepin-10(3H)-on

Neben den oder anstelle der oben verwendeten Pflanzenextrakte, die eine Mehrzahl von Inhaltsstoffen enthalten, können auch die Hauptbestandteile (einer oder mehrere) verwendet werden.

Für die oben aufgeführten Zusammensetzungen, insbesondere jene mit 5% Wirkstoffmischung, wurde in ersten Versuchen anti-virale, anti-fungale und anti-bakterielle Wirkung gefunden.

**Beispiel 2:** Das Bsp. 1 wurde wiederholt, jedoch wurde Artemisinin-Extrakt durch das Artemisinin-Isoloat (HPLC-Reinheit > 99%) ersetzt. Es werden ebeenfalls in ersten Versuchen anti-virale, anti-fungale und anti-bakterielle Wirkung gefunden.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Mizellen enthaltende Wirkstoffzusammensetzung, wobei die Mizellen enthaltende Wirkstoffzusammensetzung mindestens zwei voneinander verschiedene Typen von Mizellen, vorzugsweise in einer wässrigen Phase, enthält; und
jeder Typ von Micellen einen Kern aufweist - dieser enthält vorwiegend den Wirkstoff - und eine einfache oder komplexe Hülle aufweist - diese weist Hilfsstoffe aus der Gruppe der Polyelektrolyten und / oder Glycerinester auf - ; und
jeder Typ von Mizellen einen optisch bestimmten mittleren geometrischen Durchmesser d₉₀ von 5 - 100 nm aufweist; und
der erste Typ von komplexen Micellen:
• mindestens einen Wirkstoff enthält, und
• mindestens einen Polyelektrolyten, ausgewählt aus Oligosacchariden und/oder Polysacchariden, und
• mindestens einen Glycerinester, ausgewählt aus Fettsäureglycerinestern und/oder Glycerinestern von Wurzelharzen, enthält;
der zweite Typ von einfachen Micellen:
• mindestens einen Wirkstoff enthält, und entweder
• mindestens einen Polyelektrolyten, ausgewählt aus Oligosacchariden und/oder Polysacchariden, oder
• mindestens einen Glycerinester, ausgewählt aus Fettsäureglycerinestern und/oder Glycerinestern von Wurzelharzen, enthält.

2. Mizellen enthaltende Wirkstoffzusammensetzung gemäss Anspruch 1, **dadurch gekennzeichnet, dass**
• in jedem Typ von Mizellen der Wirkstoff und der mindestens eine Glycerinester und/oder der mindestens eine Polyelektrolyt ähnliche physikalisch-chemische Eigenschaften aufweisen; und / oder
• das Verhältnis von erstem Typ zu zweitem Typ im Bereich 1:99 bis 99:1 liegt (n/n) bevorzugt 10:90 bis 90:10, wie bspw. 2:1 bis 1:2.

3. Mizellen enthaltende Wirkstoffzusammensetzung gemäss Anspruch 1 oder 2, worin polare oder ionische Wirkstoffe enthaltende Mizellen > 50 Gew-% Polyelektrolyt, bezogen auf die Gesamtmenge an Polyelektrolyt und Glycerinester, enthalten.

4. Mizellen enthaltende Wirkstoffzusammensetzung gemäss einem der vorangehenden Ansprüche, **dadurch gekennzeichnet, dass** der pH-Wert
• unter dem pKa-Wert des Polyelektrolyten, und
• unter dem pKa-Wert eines in deprotonierter Form ionisch vorliegenden Wirkstoffs liegt.

5. Mizellen enthaltende Wirkstoffzusammensetzung gemäss einem der vorangehenden Ansprüche, **dadurch gekennzeichnet, dass** der mindestens eine Polyelektrolyt ausgewählt ist aus der Gruppe umfassend Gummi Arabicum, eine Fraktion aus Gummi Arabicum, insbesondere eine Fraktion mit erhöhtem Galakturonsäure-Gehalt, Cyclodextrin, Glykane, Glykogene, Glycolipide oder eine Mischung aus einem oder mehreren der genannten Polyelektrolyte insbesondere aber Gummi Arabicum, Cyclodextrine oder eine Mischung derselben.

6. Mizellen enthaltende Wirkstoffzusammensetzung gemäss einem der vorangehenden Ansprüche, **dadurch gekennzeichnet, dass**
• der Polyelektrolyt Gummi Arabicum oder eine Fraktion desselben ist, inbesondere mit einem pKa-Wert der Seitenketten von 4.4 bis 4.93, und
• der pH < 4 ist, insbesondere < 3.5, speziell bevorzugt ca. 3.2.

7. Mizellen enthaltende Wirkstoffzusammensetzung gemäss einem der vorangehenden Ansprüche, **dadurch gekennzeichnet, dass** der mindestens eine Glycerinester ausgewählt ist aus der Gruppe bestehend aus Monoglyceriden von Fettsäuren, Diglyceriden von Fettsäuren, Monoglyceriden von Fettsäuren und mindestens einer weiteren Säure, Diglyceriden von Fettsäuren und einer weiteren Säure, Monoglyceriden von Wurzelharzen, Diglyceriden von Wurzelharzen, Monoglyceriden von Wurzelharzen und mindestens einer weiteren Säure, Diglyceriden von Wurzelharzen und einer weiteren Säure und Mischungen aus einem oder mehreren der genannten Glycerinester, insbesondere Monoglyceriden von Fettsäuren, Diglyceriden von Fettsäuren, Monoglyceriden von Fettsäuren und mindestens einer weiteren Säure, Diglyceriden von Fettsäuren und einer weiteren Säure, wobei die Fettsäuren vorzugsweise natürliche Fettsäuren sind, sowie Monoglyceriden von Wurzelharzen, Diglyceriden von Wurzelharzen und Mischungen aus einem oder mehreren der genannten Glycerinester.

8. Mizellen enthaltende Wirkstoffzusammensetzung gemäss einem der vorangehenden Ansprüche, **dadurch gekennzeichnet, dass** der optisch bestimmte mittlere geometrische Durchmesser der Mizellen d₉₀ 10 nm - 30 nm beträgt.

9. Mizellen enthaltende Wirkstoffzusammensetzung, gemäss einem der vorangehenden Ansprüche, **dadurch gekennzeichnet, dass** die Gewichts-Verhältnisse von Wirkstoff(en) zur Summe aus Polyelektrolyt und/oder Glycerinester im Bereich von 4 : 1 bis 1 : 4 liegen.

10. Mizellen enthaltende Wirkstoffzusammensetzung gemäss einem der vorangehenden Ansprüche, **dadurch gekennzeichnet, dass** der bzw. die Wirkstoffe ausgewählt sind
• aus der Gruppe umfassend Oligopeptiden, Polypeptiden, Proteinen (insbesondere Proteine mit weniger als 30 Aminosäuren), und Antikörpern; oder
• aus der Gruppe umfassend Oligonucleotiden und Polynukleotiden; oder
• aus der Gruppe bestehend aus Curcumin, Olibanum, Artemisinin und Vitamin C und insbesondere Curcumin, Olibanum, Vitamin C und gegebenenfalls Artemisinin enthalten oder daraus bestehen.

11. Mizellen enthaltende Wirkstoffzusammensetzung gemäss einem der vorangehenden Ansprüche
• zur Behandlung viraler, bakterieller oder fungaler Infekte, insbesondere viraler Infekte; oder
• zur Behandlung von proliverativen Erkrankungen, insbesondere von Krebserkrankungen.

12. Verfahren zur Herstellung einer Zusammensetzung nach Anspruch 1, umfassend
(A) Herstellung eines Typs von Mizellen in Form einer mizellaren Phase oder einer mizellaren Dispersion, umfassend:
einen ersten Schritt, in dem unter Mischen mindestens ein Polysaccharid und gegebenenfalls mindestens ein Glycerinester getrocknet werden und das mindestens eine Polysaccharid anschliessend bei einer ersten Temperatur geschmolzen oder durch Zugabe mindestens eines Glycerinesters gelöst wird,
einen zweiten Schritt, in dem der Wirkstoff bei der ersten Temperatur zugemischt und die Mizellenbildung vorbereitet wird, indem gewartet wird, bis die durch Zugabe des Wirkstoffs trüb gewordene Zusammensetzung wieder klar wird,
einen dritten Schritt, in dem zur klaren Zusammensetzung aus dem zweiten Schritt unter Mischen Lösungsmittel, insbesondere Wasser oder eine wässrige Lösung, vorzugsweise vorbestimmten pH-Werts, zugegeben, wird, worauf sich selbstregulierend sphärische Formen, in Form einer micellaren Phase oder micellaren Dispersion bilden; und
(B) Vermischen von zwei oder mehr dieser mizellaren Phasen und/oder mizellaren Dispersionen.

13. Das Verfahren gemäss Anspruch 12, **dadurch gekennzeichnet, dass**
im ersten Schritt (A) die Trocknung durch Erhitzen, gegebenenfalls und bevorzugt unter Vakuum, erfolgt und/oder die Temperatur und der Gasdruck so gewählt werden, dass die Polyelektrolyte und/oder Glycerinester vollkommen verflüssigt und auf konstanter Temperatur sind, und/oder
im zweiten Schritt (A) die Zusammensetzung nach Zugabe des Wirkstoffs entgast wird, und/oder
im dritten Schritt (A) zugegebenes Wasser oder zugegebene wässrige Lösung zuvor auf einen bestimmten pH-Wert eingestellt wird, derart, dass in deprotonierter Form ionischer Wirkstoff und Polysaccharid beide mehrheitlich protoniert oder deprotoniert vorliegen und/oder die Temperatur des Wassers oder der wässrigen Lösung auf eine zweite Temperatur eingestellt wird, die der ersten Temperatur, d.h. der Temperatur der Zusammensetzung aus dem zweiten Schritt, minus maximal 15°C entspricht; und / oder
im Schritt (B) die Ionenstärke, pH - Wert und Micellengrösse besagter mizellaren Phasen und/oder besagter mizellaren Dispersionen +/- 10 des jeweiligen Parameters sind.

14. Feste Darreichungsform umfassend eine Mizellen enthaltende Wirkstoffzusammensetzung gemäss einem der Ansprüche 1 - 11

15. Feste Darreichungsform nach Anspruch 14, ausgewählt aus Dragees oder Kapseln.

## Claims

1. Active ingredient composition containing micelles,
wherein
the micelle-containing active ingredient composition comprises at least two different types of micelles, preferably in an aqueous phase; and
each type of micelle has a core - which predominantly contains the active ingredient - and a simple or complex shell - which contains auxiliary substances from the group of polyelectrolytes and/or glycerol esters - ; and
each type of micelle has an optically determined mean geometric diameter d₉₀ of 5 - 100 nm; and
the first type of complex micelles:
• contains at least one active substance, and
• at least one polyelectrolyte selected from oligosaccharides and/or polysaccharides, and
• contains at least one glycerol ester selected from fatty acid glycerol esters and/or glycerol esters of root resins;
the second type of simple micelles:
• contains at least one active substance, and either
• at least one polyelectrolyte selected from oligosaccharides and/or polysaccharides, or
• contains at least one glycerol ester selected from fatty acid glycerol esters and/or glycerol esters of root resins.

2. Active ingredient composition containing micelles according to claim 1, **characterised in that**
• in each type of micelles, the active ingredient and the at least one glycerol ester and/or the at least one polyelectrolyte have similar physical-chemical properties; and/or
• the ratio of first type to second type is in the range 1:99 to 99:1 (n/n) preferably 10:90 to 90:10, such as 2:1 to 1:2.

3. Active ingredient composition containing micelles according to claim 1 or 2, wherein micelles containing polar or ionic active ingredients contain > 50% by weight of polyelectrolyte, based on the total amount of polyelectrolyte and glycerol ester.

4. Active ingredient composition containing micelles according to one of the preceding claims, **characterised in that** the pH value
• is below the pKa value of the polyelectrolyte, and
• is below the pKa value of an active ingredient in deprotonated ionic form.

5. Active ingredient composition containing micelles according to one of the preceding claims, **characterised in that** the at least one polyelectrolyte is selected from the group comprising gum arabic, a fraction of gum arabic, in particular a fraction with an increased galacturonic acid content, cyclodextrin, glycans, glycogenes, glycolipids or a mixture of one or more of the said polyelectrolytes, but in particular gum arabic, cyclodextrins or a mixture thereof.

6. Active ingredient composition containing micelles according to one of the preceding claims, **characterised in that**
• the polyelectrolyte is gum arabic or a fraction thereof, in particular with a pKa value of the side chains of 4.4 to 4.93, and
• the pH is < 4, in particular < 3.5, especially preferably approx. 3.2.

7. Active ingredient composition containing micelles according to one of the preceding claims, **characterised in that** the at least one glycerol ester is selected from the group consisting of monoglycerides of fatty acids, diglycerides of fatty acids, monoglycerides of fatty acids and at least one further acid, diglycerides of fatty acids and a further acid, monoglycerides of root resins, diglycerides of root resins, monoglycerides of root resins and at least one further acid, diglycerides of root resins and a further acid and mixtures of one or more of the said glycerol esters, in particular monoglycerides of fatty acids, diglycerides of fatty acids, monoglycerides of fatty acids and at least one further acid, diglycerides of fatty acids and a further acid, the fatty acids preferably being natural fatty acids, and monoglycerides of root resins, diglycerides of root resins and mixtures of one or more of the said glycerol esters.

8. Active ingredient composition containing micelles according to one of the preceding claims, **characterised in that** the optically determined mean geometric diameter of the micelles d₉₀is 10 nm - 30 nm.

9. Active ingredient composition containing micelles, according to one of the preceding claims, **characterised in that** the weight ratios of active ingredient(s) to the sum of polyelectrolyte and/or glycerol ester are in the range from 4:1 to 1:4.

10. active ingredient composition containing micelles according to one of the preceding claims, **characterised in that** the active ingredient(s) are selected from
• from the group comprising oligopeptides, polypeptides, proteins (in particular proteins with less than 30 amino acids), and antibodies; or
• selected from the group consisting of oligonucleotides and polynucleotides; or
• from the group consisting of curcumin, olibanum, artemisinin and vitamin C and in particular containing or consisting of curcumin, olibanum, vitamin C and optionally artemisinin.

11. active ingredient composition containing micelles according to one of the preceding claims
• for the treatment of viral, bacterial or fungal infections, especially viral infections; or
• for the treatment of proliferative diseases, especially cancer.

12. Process for the preparation of a composition according to claim 1, comprising
(A) Preparation of a type of micelles in the form of a micellar phase or a micellar dispersion comprising:
a first step in which at least one polysaccharide and optionally at least one glycerol ester are dried with mixing and the at least one polysaccharide is subsequently melted at a first temperature or dissolved by addition of at least one glycerol ester,
a second step in which the active ingredient is added at the first temperature and the micelle formation is prepared by waiting until the composition that has become cloudy due to the addition of the active ingredient becomes clear again,
a third step in which solvent, in particular water or an aqueous solution, preferably of predetermined pH, is added to the clear composition from the second step with mixing, whereupon self-regulating spherical moulds are formed in the form of a micellar phase or micellar dispersion; and
(B) mixing of two or more of these micellar phases and/or micellar dispersions.

13. The method according to claim 12, **characterised in that**
in the first step (A), the drying is carried out by heating, optionally and preferably under vacuum, and/or the temperature and the gas pressure are selected such that the polyelectrolytes and/or glycerol esters are completely liquefied and at a constant temperature, and/or
in the second step (A), the composition is degassed after addition of the active ingredient, and/or
water or aqueous solution added in the third step (A) is previously adjusted to a specific pH value such that in deprotonated form the ionic active ingredient and polysaccharide are both predominantly protonated or deprotonated and/or the temperature of the water or aqueous solution is adjusted to a second temperature which corresponds to the first temperature, i.e. the temperature of the composition from the second step, minus a maximum of 15°C; and/or
in step (B), the ionic strength, pH value and micelle size of said micellar phases and/or of said micellar dispersions are +/- 10 of the respective parameter.

14. A solid dosage form comprising a micelle-containing active ingredient composition according to any one of claims 1-11.

15. The solid dosage form according to claim 14, selected from coated tablets or capsules.

## Revendications

1. Composition active contenant des micelles, dans laquelle la composition de principe actif contenant des micelles contient au moins deux types de micelles différents l'un de l'autre, de préférence dans une phase aqueuse ; et
chaque type de micelles présente un noyau - celui-ci contient principalement le principe actif - et une enveloppe simple ou complexe - celle-ci présente des adjuvants du groupe des polyélectrolytes et/ou des esters de glycérol - ; et
chaque type de micelle a un diamètre géométrique moyen d₉₀déterminé optiquement de 5 à 100 nm ; et
le premier type de micelles complexes :
• contient au moins une substance active, et
• au moins un polyélectrolyte choisi parmi les oligo-saccharides et/ou les polysaccharides, et
• contient au moins un ester de glycérol choisi parmi les esters de glycérol d'acides gras et/ou les esters de glycérol de résines de racines ;
le deuxième type de micelles simples :
• contient au moins une substance active, et soit
• au moins un polyélectrolyte choisi parmi les oligo-saccharides et/ou les polysaccharides, ou
• contient au moins un ester de glycérol choisi parmi les esters de glycérol d'acides gras et/ou les esters de glycérol de résines de racines.

2. Composition de principe actif contenant des micelles selon la revendication 1, **caractérisée en ce que**
• dans chaque type de micelles, le principe actif et le au moins un ester de glycérol et/ou le au moins un polyélectrolyte présentent des propriétés physico-chimiques similaires ; et/ou
• le rapport entre le premier type et le deuxième type se situe dans la plage de 1:99 à 99:1 (n/n), de préférence de 10:90 à 90:10, comme par exemple de 2:1 à 1:2.

3. Composition de principes actifs contenant des micelles selon la revendication 1 ou 2, dans laquelle les micelles contenant des principes actifs polaires ou ioniques contiennent > 50 % en poids de polyélectrolyte, par rapport à la quantité totale de polyélectrolyte et d'ester de glycérol.

4. Composition de principe actif contenant des micelles selon l'une des revendications précédentes, **caractérisée en ce que** le pH
• inférieure au pKa du polyélectrolyte, et
• est inférieure à la valeur pKa d'une substance active présente sous forme ionique déprotonée.

5. Composition de principe actif contenant des micelles selon l'une des revendications précédentes, **caractérisée en ce que** le au moins un polyélectrolyte est choisi dans le groupe comprenant la gomme arabique, une fraction de gomme arabique, en particulier une fraction à teneur accrue en acide galacturonique, la cyclodextrine, les glycanes, les glycogènes, les glycolipides ou un mélange d'un ou plusieurs des polyélectrolytes mentionnés, en particulier mais pas la gomme arabique, les cyclodextrines ou un mélange de ceux-ci.

6. Composition de principe actif contenant des micelles selon l'une des revendications précédentes, **caractérisée en ce que**
• le polyélectrolyte est la gomme arabique ou une fraction de celle-ci, en particulier avec une valeur de pKa des chaînes latérales de 4,4 à 4,93, et
• le pH est < 4, en particulier < 3,5, de préférence environ 3,2.

7. Composition de principe actif contenant des micelles selon l'une des revendications précédentes, **caractérisée en ce que** ledit au moins un ester de glycérol est choisi dans le groupe constitué par les monoglycérides d'acides gras, les diglycérides d'acides gras, les monoglycérides d'acides gras et au moins un autre acide, les diglycérides d'acides gras et un autre acide, les monoglycérides de résines de racine, les diglycérides de résines de racine, les monoglycérides de résines de racine et au moins un autre acide, des diglycérides de résines de racines et d'un autre acide et des mélanges d'un ou de plusieurs desdits esters de glycérol, en particulier des monoglycérides d'acides gras, des diglycérides d'acides gras, des monoglycérides d'acides gras et d'au moins un autre acide, des diglycérides d'acides gras et d'un autre acide, les acides gras étant de préférence des acides gras naturels, ainsi que des monoglycérides de résines de racines, des diglycérides de résines de racines et des mélanges d'un ou de plusieurs desdits esters de glycérol.

8. Composition de principe actif contenant des micelles selon l'une des revendications précédentes, **caractérisée en ce que** le diamètre géométrique moyen des micelles, déterminé optiquement, est d₉₀10 nm - 30 nm.

9. Composition de principe actif contenant des micelles, selon l'une des revendications précédentes, **caractérisée en ce que** les rapports pondéraux du ou des principes actifs à la somme du polyélectrolyte et/ou de l'ester de glycérine se situent dans la plage de 4 : 1 à 1 : 4.

10. Composition micellaire de principes actifs selon l'une quelconque des revendications précédentes, **caractérisée en ce que** le ou les principes actifs sont choisis parmi les suivants
• du groupe comprenant les oligopeptides, les polypeptides, les protéines (en particulier les protéines de moins de 30 acides aminés), et les anticorps ; ou
• du groupe comprenant des oligonucléotides et des polynucléotides ; ou
• du groupe constitué par la curcumine, l'oliban, l'artémisinine et la vitamine C et, en particulier, contiennent ou sont constitués de curcumine, d'oliban, de vitamine C et, le cas échéant, d'artémisinine.

11. composition active contenant des micelles selon l'une des revendications précédentes
• pour le traitement d'infections virales, bactériennes ou fongiques, en particulier les infections virales ; ou
• pour le traitement des maladies prolixes, en particulier des cancers.

12. procédé de préparation d'une composition selon la revendication 1, comprenant
(A) la préparation d'un type de micelles sous la forme d'une phase micellaire ou d'une dispersion micellaire, comprenant :
une première étape dans laquelle on sèche en mélangeant au moins un polysaccharide et éventuellement au moins un ester de glycérol, puis on fait fondre ledit au moins un polysaccharide à une première température ou on le dissout en ajoutant au moins un ester de glycérol,
une deuxième étape au cours de laquelle le principe actif est mélangé à la première température et la formation de micelles est préparée en attendant que la composition, devenue trouble par l'ajout du principe actif, redevienne claire,
une troisième étape dans laquelle on ajoute à la composition claire de la deuxième étape, en mélangeant, un solvant, en particulier de l'eau ou une solution aqueuse, de préférence de valeur de pH prédéterminée, après quoi il se forme de manière autorégulée des formes sphériques, sous la forme d'une phase micellaire ou d'une dispersion micellaire ; et
(B) mélanger deux ou plusieurs desdites phases micellaires et/ou dispersions micellaires.

13. Le procédé selon la revendication 12, **caractérisé en ce que**
dans la première étape (A), le séchage est effectué par chauffage, éventuellement et de préférence sous vide, et/ou la température et la pression du gaz sont choisies de manière à ce que les polyélectrolytes et/ou les esters de glycérol soient totalement liquéfiés et à une température constante, et/ou
dans la deuxième étape (A), la composition est dégazée après l'ajout de la substance active, et/ou
l'eau ou la solution aqueuse ajoutée dans la troisième étape (A) est préalablement ajustée à une valeur de pH déterminée, de telle sorte que, sous forme déprotonée, la substance active ionique et le polysaccharide se présentent tous deux majoritairement protonés ou déprotonés et/ou la température de l'eau ou de la solution aqueuse est ajustée à une deuxième température qui correspond à la première température, c'est-à-dire à la température de la composition issue de la deuxième étape, moins 15°C au maximum ; et / ou
dans l'étape (B), la force ionique, le pH et la taille des micelles desdites phases micellaires et/ou desdites dispersions micellaires sont +/- 10 du paramètre respectif.

14. Forme galénique solide comprenant une composition de principe actif contenant des micelles selon l'une quelconque des revendications 1 à 11.

15. Forme galénique solide selon la revendication 14, choisie parmi les dragées ou les capsules.
